# EUROPEAN PATENT APPLICATION

(11) **EP 4 806 361 A1**
(43) Date of publication of application: **16.09.2026**
(21) Application number: 26164438.9
(22) Date of filing: 12.03.2026
(51) Int. Cl.: A61B 6/03, A61B 6/51, A61B 6/00

(54) **GENERATION OF MAXILLOFACIAL IMAGING VOLUME WITH MOTION**

(30) Priority: 12.03.2025 US 202563770626 P; 23.02.2026 US 202619547442
(71) Applicant: Hülsbusch, Markus, 68642 Bürstadt (DE); Maur, Susanne, 64625 Bensheim (DE); Beckhaus, Christian, 64720 Michelstadt (DE)
(72) Inventor: Hülsbusch, Markus, 68642 Bürstadt (DE); Maur, Susanne, 64625 Bensheim (DE); Beckhaus, Christian, 64720 Michelstadt (DE)
(74) Representative: Aldridge, Henry Alexander

(57) **Abstract**

Various aspects of systems, methods, and related aspects to generate a maxillofacial imaging volume with motion, from a three-dimensional (3D) extra-oral x-ray system, are described. One example technique includes: obtaining imaging data of a patient, captured during movement of an upper jaw and the lower jaw of the patient; establishing a motion model that defines an expected movement of the upper jaw relative to the lower jaw; segmenting the imaging data into at least an upper jaw segment corresponding to the upper jaw and a lower jaw segment corresponding to the lower jaw, based on the expected movement defined by the motion model; performing motion artifact correction separately on the upper jaw segment and the lower jaw segment; and outputting an imaging volume that includes an artifact-corrected representation of the upper jaw segment and an artifact-corrected representation of the lower jaw segment.

## Description

### PRIORITY CLAIM

This application claims the benefit of priority to United States Provisional Patent Application No. 63/770,626, filed March 12, 2025, and titled "GENERATION OF MAXILLOFACIAL IMAGING VOLUME WITH MOTION", which is incorporated herein by reference in its entirety.

### BACKGROUND

In some settings, jaw movement may need to be captured and portrayed in connection with dental and maxillofacial diagnostic imaging procedures. For example, the movement of a patient's jaw and the range of motion that is experienced from chewing or biting may be relevant to the diagnosis or treatment of a dental condition.

Some limited techniques are used today to measure or replicate jaw motion. One existing technique includes the capture of static cone-beam computed tomography (CBCT) and digital intraoral images (e.g., obtained with an intraoral optical scanner) that portray different positions of the patient's jaw, such as images captured with a first scan when the patient's jaw is opened and a second scan when the patient's jaw is closed. This is combined with data captured by a mechanical articulator that provides sensors to measure movement and jaw positions. The matching of movements observed by a mechanical articulator to the positions captured in the digital imaging can be time-consuming and introduces the potential of many sources of error.

Another existing technique to replicate jaw motion includes the use of a virtual articulator, which involves software that processes CBCT (and optionally, optical scan images) to create a virtual representation that models the relationship between the upper and lower jaws and simulates movement of each jaw. The use of a virtual articulator, however, often involves the manual modification and definition of parameters of the jaw movement to properly position and operate the jaw models in a virtual space. As a result, the use of mechanical and virtual articulators often relies on human modification and may not fully model the correct range of motion of a patient's temporomandibular joints.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
FIG. 1 depicts a computerized extraoral x-ray system, according to an example.
FIG. 2 depicts a flowchart of an algorithm for capturing images and outputting representations of a patient jaw, according to an example.
FIG. 3 depicts aspects of segmentation operations used with images of patient jaw movement, according to an example.
FIG. 4 depicts aspects of motion artifact correction operations used with images of patient jaw movement, according to an example.
FIG. 5 depicts aspects of displaying corrected representations of jaw segments, according to an example.
FIG. 6 depicts an example segmentation of jaw segments, according to an example.
FIG. 7 depicts a flowchart of an example method for generating an imaging volume of a maxillofacial region with movement, according to an example.
FIG. 8 depicts a block diagram of an example of a computing system upon which any one or more of the techniques discussed herein may be performed, according to an example.

### DETAILED DESCRIPTION

The following is directed to improvements in image processing technologies to capture a CBCT scan with intentional movement, such as a chewing movement or jaw opening and closing movement. Specifically, the following approaches introduce techniques to generate a motion-corrected 3D imaging volume, while also performing a functional evaluation of the motion performed during the image capture. This includes the correction of non-rigid movements through several, rigidly assumed movements of partial volumes, such as a respective volume defined for the upper jaw and the lower jaw. As used herein, the imaging of an "upper jaw" refers to imaging of the maxillae and all rigidly connected structures that are rigid themselves, such as the upper set of teeth, crowns, implants, etc., and the imaging of a "lower jaw" refers to imaging of the mandible and all rigidly connected structures that are rigid themselves such as the lower set of teeth, crowns, implants, etc.

The accurate capture and playback of motion can be very difficult in many types of imaging procedures because the motion that occurs during x-rays or CBCT scans will introduce a number of artifacts that degrade the underlying images. With the following approaches, motion artifact correction (MAC) is performed together with a segmentation process, as the segmentation process separates the upper and lower portions of the jaw and converts each of the portions into rigidly assumed sub-volumes (e.g., a respective volume for the upper and lower jaw). These sub-volumes, defining smaller sections of the overall scanned volume, are basically treated as if they do not deform or change shape during the image scanning.

The segmentation process may be performed on a sinogram (e.g., a representation of a raw projection image acquired during the x-ray or CBCT scan) or on a reconstructed volume (e.g., the 3D representation that is reconstructed from the x-ray or CBCT projection images). A functional analysis of the movement may be performed during the motion artifact correction, such as with the use of a parameterized motion model, or the functional analysis of the movement may be performed after completing the motion artifact correction. As used herein, a "motion model" refers to a computerized representation of jaw movement characteristics, which is used for determining expected motion (e.g., a determination of how a patient's jaw is expected to move such as during imaging) and estimated motion (e.g., a determination of how a patient's jaw has actually moved during the imaging). This motion model can be instantiated with specific parameters (e.g., values or settings of the model) for a specific patient, to help model the physical joint behavior for the specific patient based on incomplete information from patient-specific imaging.

In some examples, the parameterized motion model may correspond to an articulator model. The relative (rigid) motion between the upper and lower jaw may be mathematically described by three translation parameters and three rotation parameters, which vary over time. However, due to anatomical constraints, this flexible jaw movement is not possible. An articulator model may limit the degrees of freedom of the motion to a rotation around an axis, which may also slightly change position during jaw opening. This axis should pass through both temporomandibular joints. Thus, the parameterization of the presently described motion model can include a rotation angle around an axis and a position of the rotation axis.

The presently described motion model may be implemented with different parameters to represent jaw movement. As an example, the following three implementations of a motion model can be used separately, mixed, or the respective motion models can be transformed into each other.

In a first implementation, the motion model includes a minimum and maximum opening angle of the jaw. The model describes a relation between the opening angle and the relative position of the jaws. The model can include a relative assumption of the speed of a jaw movement related to a sequence of opening states / jaw movement (e.g., speed up, slow down during chewing).

In a second implementation, the motion model is based on anatomical conditions, such as the position and size or shape or other properties of the joints, describing the jaw's possibility to move.

In a third implementation, the motion model is provided with basic features, but many parameters. The position of each jaw can be described over time with rotation and translation. This can include three rotation and three translation parameters (or less) per point in time or per opening angle.

Various aspects of these motion models can be refined. When a motion model is refined, updates are made to parameters such as the minimum or maximum opening angle, the relation between the opening angle and the relative position of the jaws, the typical speed, anatomical conditions of the joints, etc. This refinement allows the motion model to better describe the estimated motion (e.g., to describe with less error) and/or to better fit the image data (e.g., to fit with less error) and/or to keep the deviation from a standard model small, because patient anatomy and movement are expected to follow some norms.

The approaches discussed herein can be used to accurately capture and digitally reproduce an imaging volume that portrays the unique movement of a patient's jaw. This may be useful in a variety of diagnostic and treatment contexts, including but not limited to: reviewing the temporomandibular joints during movement; creating a bite splint; performing an examination of temporomandibular joint pain; or checking the opposing bite when creating dentures.

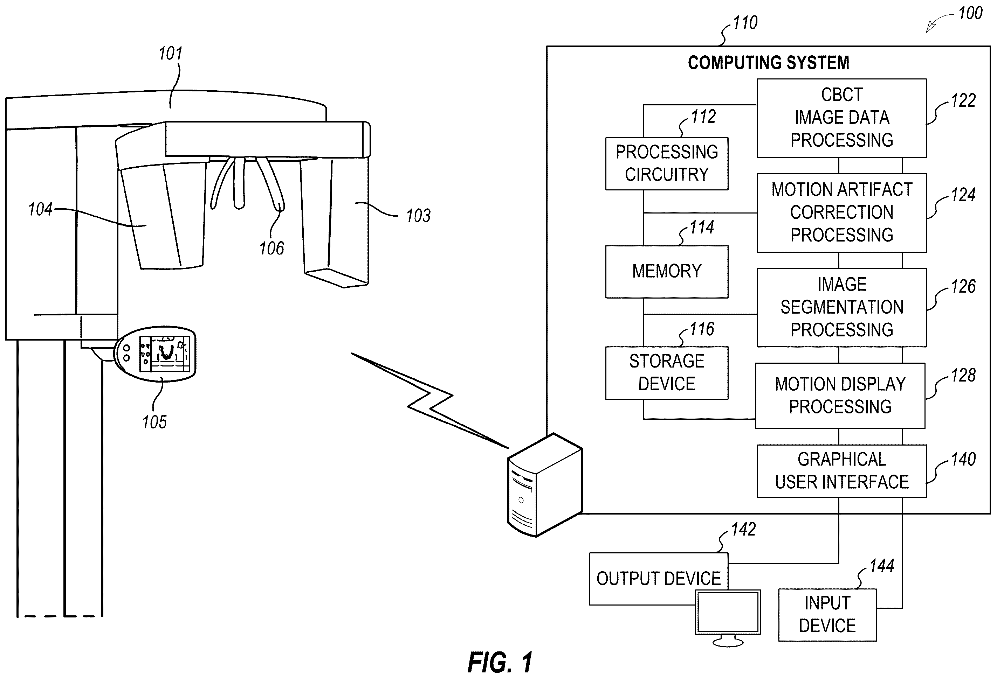

FIG. 1 depicts a computerized extraoral x-ray system 100 usable with the present techniques. As shown in FIG. 1, the x-ray system 100 includes an x-ray device 101 for performing the patient imaging. Prior to the exposure, the patient's head (not shown) is preferably positioned in a center of the x-ray device 101, such as with a head fixation 106. As discussed below, the x-ray device 101 includes an x-ray emitter 103 and an x-ray detector 104 that rotate around the patient head to obtain x-ray images from different angles. The x-ray device 101 may include an operator device 105 that includes a basic user interface or operator control (e.g., touch screen, buttons, etc.), to activate and control various functions of the x-ray system 100. The x-ray system 100 may be capable of producing 2D and 3D x-ray images, including with the use of CBCT x-ray beams, with the resulting images being reconstructed to generate a 3D volume of a craniofacial region and specifically a maxillofacial region of a patient. As used herein, the term "imaging data" refers to projection images (e.g., produced from an x-ray detector), sinograms (e.g., produced from the projections), reconstructed imaging volumes (e.g., produced from a reconstruction process using the projection images or the sinograms), or any combination thereof.

As also shown in FIG. 1, the x-ray system 100 may operably connect the x-ray device 101 with a separate computing system 110 (or set of computers) and a separate output device 142 (such as a display device) to visualize data sets. The computing system 110 can be connected to the x-ray device 101 via a local network (not shown) or alternatively via the Internet. The computing system 110 may be part of a cloud computing deployment. In other examples, functions of the computing system 110 may be integrated into the x-ray device 101. Thus, the data processing operations described herein may take place on the x-ray device 101, on the computing system 110, or in a connected computing resource in the cloud.

For example, the computing system 110 may execute various computer programs and manipulate data sets to create different visualizations and representations of imaging data. In some examples, the computing system 110 may provide commands to control the x-ray device 101 via the input device 144 or via programmatic control. Alternatively, separate computers may be used for image processing and device control operations. The imaging data sets generated herein may be presented to a clinician (e.g., physician, dentist, trained staff, etc.) for visualization, in particular for diagnostic purposes, by use of the output device 142 or a communication to another type of output device such as a printer, screen, etc.

The x-ray system 100 is adapted for performing dental imaging procedures for evaluative or diagnostic purposes, including for generating an imaging volume of the maxillofacial area of a patient with motion using the techniques discussed below. As shown in FIG. 1, the x-ray system 100 comprises an x-ray emitter 103. The x-ray device 101 further comprises an x-ray detector 104 for at least partially detecting the x-ray radiations emitted by the x-ray emitter during a revolution.

The x-ray device 101 may further include an aperture mechanism (not shown) for collimating the x-ray radiation emitted by the x-ray emitter onto the respective area to be irradiated. The x-ray device 101 may further include a control device or mechanism (not shown) for moving the x-ray emitter 103 and the x-ray detector 104, and for controlling the x-ray emitter and for reading out the projection image series of the respective irradiated areas of the x-ray detector 104 during the rotation. These and other features may be controlled by on-board logic and processing circuitry on the x-ray device 101, which may be controlled by the operator device 105 in some examples.

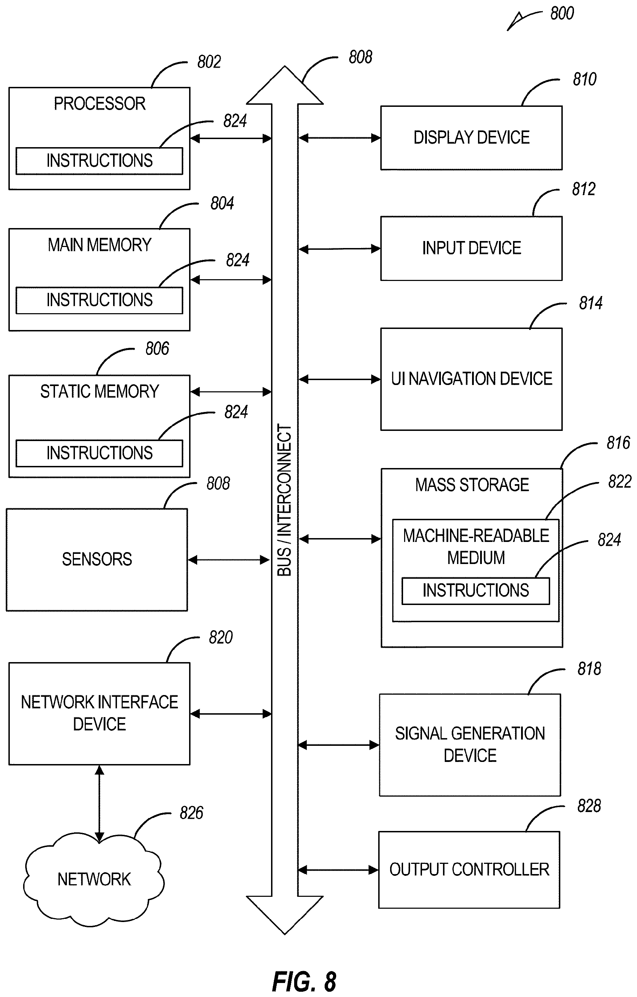

The computing system 110 is depicted as including processing circuitry 112, memory 114, and a storage device 116. The processing circuitry 112 may execute machine-readable software instructions (e.g., provided from the memory 114 or the storage device 116) to perform operations that implement CBCT image data processing 122, motion artifact correction processing 124, image segmentation processing 126, motion display processing 128, and a graphical user interface 140, as discussed below. The image processing methods discussed herein may be computer-implemented methods that can be executed via coordination of the x-ray device 101, the computing system 110, or the x-ray system 100 which includes both sub-systems. Further example implementations of the processing circuitry 112, the memory 114, and the storage device 116, are provided below with reference to FIG. 8.

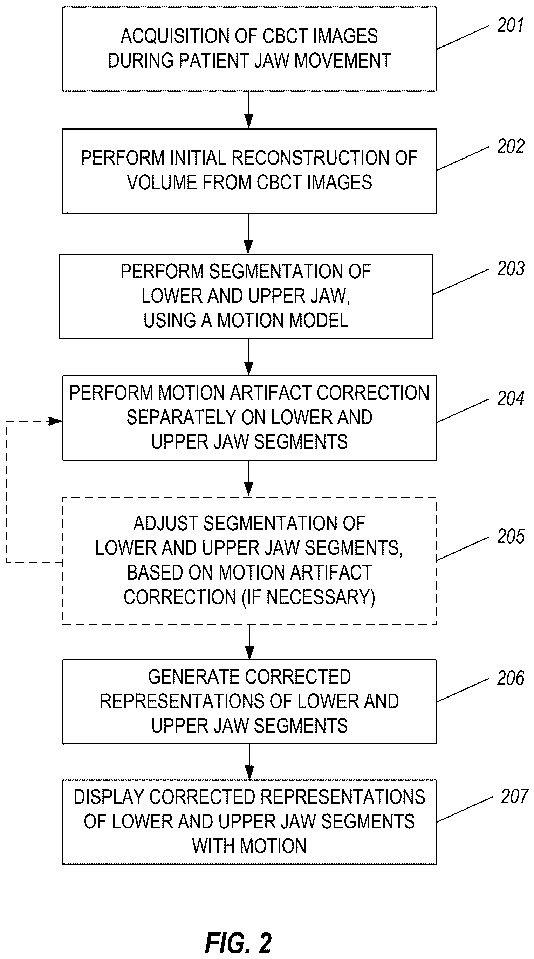

The image processing methods performed by the computing system 110 may include CBCT image data processing 122 used to capture and process CBCT projection images and reconstruct an image volume from the projection images. The motion artifact correction processing 124 is used to correct distortions caused by movement during capture of the CBCT imaging data and is used in coordination with the image segmentation processing 126 that provides a segmentation and separation of rigid anatomical structures (e.g., the upper jaw and the lower jaw) in the imaging data. The motion display processing 128 is used to generate displays and representations of patient motion, such as animated movement between two segmented image volumes that are defined for the upper jaw and the lower jaw, respectively. Further details on the creation of a CBCT image data with motion are discussed in FIGS. 2 to 7, below. The results from the motion display processing 128 can be used to create a 3D or 4D imaging volume to be presented via a graphical user interface 140, such as an image display user interface that is controlled via an input device 144 and presented via the output device 142. Other image data processing functions of the computing system 110 may be invoked via the graphical user interface 140 or the operator device 105.

The present image processing methods for capturing and reconstructing an imaging volume of a jaw with intentional, controlled motion provide significant benefits over existing approaches that use mechanical or virtual articulators. In particular, the present image processing methods enable simultaneous motion artifact correction with segmentation, which enables the correction of non-rigid movements in CBCT imaging data prior to the reconstruction of the imaging volume. This can enable simultaneous acquisition of the static and moving CBCT imaging data, in addition to a simultaneous functional acquisition and evaluation of the jaw movement. Further, the present approaches can provide a simplified workflow that provides time savings because a mechanical actuator does not need to be attached to the patient or calibrated and tracked in a system. Moreover, an accurate representation of the patient's bite can be fully simulated in a 3D image volume because a bite block does not need to be used to keep the patient's jaw stationary.

FIG. 2 depicts a flowchart of an example algorithm for capturing images and outputting representations of a patient jaw. Although this flowchart depicts a sequence of operations, some of the operations may be repeated, omitted, or performed in an alternate sequence.

Operation 201 includes the acquisition of CBCT projection imaging data during a patient's jaw movement. For example, the acquisition may include the use of an extraoral x-ray unit such as the x-ray device 101 that obtains individual CBCT projection images as the x-ray emitter rotates around the patient's head while the patient's jaw is moving. The projection imaging data acquisition may include capturing multiple 2D projection images, as multiple rotations of the x-ray device 101 are performed around a patient's head while the patient continues to make the jaw movements. In some examples, this may include capturing the CBCT images during more than two rotations, depending on the speed of the scanning and rotation, sampling rate of the projection images, and a maximum dose to deliver to the patient from the x-rays. As will be understood, the image quality of the resulting reconstructed image volume can be improved by increasing the number of projection images from the same geometry. In some examples, the CBCT images are captured during a half rotation or less than two rotations, which can lead to decreased image quality of the resulting reconstructed image volume, but which can be improved by further techniques such as sparse sampling or artificial intelligence.

Various techniques may be used to control or enhance the acquisition of the CBCT imaging data in connection with the patient's jaw movement. For example, a patient may be instructed to open and close their jaw a specific number of times or provide the motion at a certain speed or rate. Such instructions can help capture redundant data used to enhance the resulting CBCT imaging data or the functional analysis of the movement. For instance, the instructions that guide the patient's jaw movement may be signaled to the human user through the use of audible acoustic signals or displayed visual indicators (e.g., information displayed on the operator device 105 or another screen), including with signals or indicators that instruct when the patient is to open and close their mouth, how far to open the jaw, how fast to open or close the jaw, etc. Additional software-based measurements may be used to determine if the patient's jaw movement is correct or is too slow, too fast, or if the range of motion is incomplete. The measurements of the patient's jaw movements may be based on an evaluation of data from the x-ray projection images, an x-ray sinogram, or external sensing devices (e.g., a camera that captures images or video of the patient's jaw movement).

Operation 202 includes performing an initial reconstruction of a 3D image volume from the acquired CBCT images (e.g., multiple projection images from a geometry that are compiled into a sinogram). In some examples, various image correction techniques may be used before or after the reconstruction, such as dark-field correction, gain or flat-field correction, or defect correction (e.g., scatter removal, noise reduction, contrast enhancement, etc.). In other examples, such image correction techniques may be used after applying segmentation and motion artifact correction (discussed below in operations 203, 204, 205).

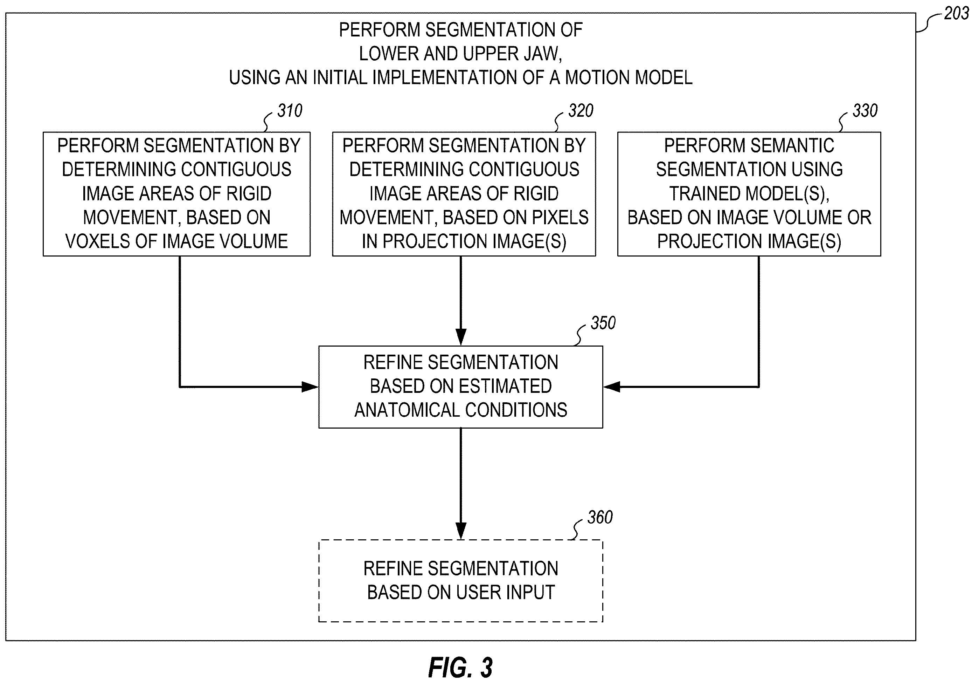

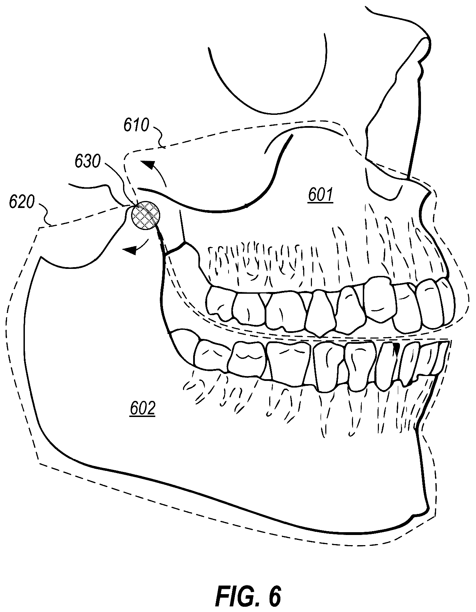

Operation 203 includes segmenting the lower jaw and the upper jaw in the reconstructed volume, using an initial implementation of a motion model. FIG. 3, discussed below, depicts example segmentation techniques applied to projection images or reconstructed image voxels, to perform and refine segmentation. When segmentation is applied to the projection images, then the motion model can provide information on where structures (e.g., the lower jaw) are to be expected in the projection image, since the position of the structures varies over time and from one projection image to the next. FIG. 6, discussed below, also depicts an example view of segmentation from a view of a reconstructed CBCT volume, including the segmentation of imaging data into an upper jaw segment corresponding to a patient's upper jaw, and a lower jaw segment corresponding to a patient's lower jaw. When segmentation is applied in an imaging volume, then the motion model can provide information on where structures (e.g., the lower jaw) are to be expected according to geometric conditions. However, an initial implementation of a motion model (e.g., the motion model together with time-varying parameters) can be used during the reconstruction of the imaging volume, which can help reduce motion artifacts of the volume and improve the segmentation results.

Various techniques may be used to determine or refine the parameters of the motion model. As a first example, the parameters of the motion model may be determined or modified from generally valid assumptions about the patient's anatomy which are parameterized from one or more landmarks detected in a sinogram. As a second example, the parameters of the motion model may be determined or refined from a guided jaw movement performed by the patient. As a third example, the parameters of the motion model may be determined or modified based on selection from several standard jaw movements. As a fourth example, the parameters of the motion model may be determined or modified based on measurements of the start and the end of the patient's movement, such as corresponding to a tracking system or a related sensor (e.g., a sensor located at a bite block). This refinement of the parameters of the motion model can result in an improved display of the jaw, because the quality of the reconstructed images will improve from more accurate motion artifact correction and segmentation operations performed with the motion model.

The motion model used in operation 203 describes a sequence of movements of the jaws, preferably relative to each other. The motion model can be used to simulate a typical jaw movement over time, or to reproduce a movement observed in image data. The motion model also can be used to describe the result of the motion artifact correction, such as by describing a sequence of movements for the image data. Further, the motion model can be refined to match the observed movement or anatomical conditions in image data.

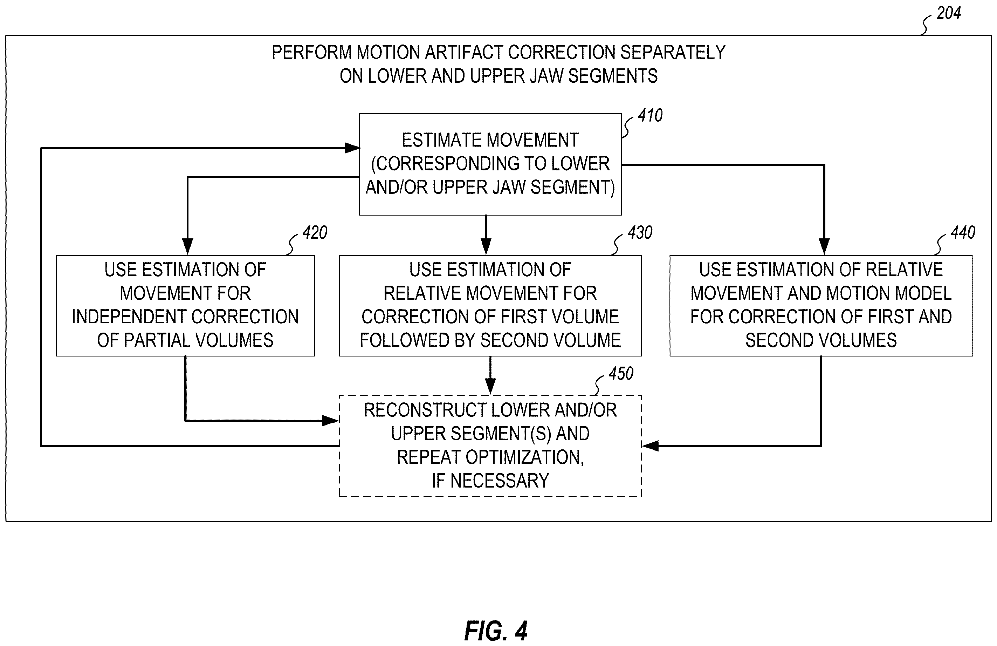

Operation 204 includes performing motion artifact correction separately on the lower and upper jaw segments, produced from the segmentation of operation 203. FIG. 4, discussed below, depicts example motion artifact correction techniques applied to the lower and upper jaw segments. These motion artifact correction techniques may be modified or adapted based on the expected jaw motion indicated by the motion model. Additionally, as discussed below, the motion artifact correction techniques may be performed on the partial volumes corresponding to the respective jaw segments independently, sequentially, or concurrently. The motion artifact correction may focus on the correction of rigid motion.

In some examples, the motion model can be used to describe the movement in the image data during the motion artifact correction. This limits the possible movements to a probable set or range of movements and therefore facilitates the motion artifact correction. Accordingly, the motion model may be used to reduce the number of possible movements, reduce the number or complexity of parameters to optimize, or provide a weight to determine possible movements.

Operation 205 includes an optional step of adjusting the segmentation of the lower and upper jaw segments based on the results of the motion artifact correction. Operation 450 in FIG. 4 provides an optional example of how the upper and/or lower jaw segments may be reconstructed into refined segments and motion artifact correction can be repeated on the refined segments, if necessary.

Operation 206 includes generating motion artifact-corrected representations of the lower and upper jaw segments. These motion artifact-corrected representations of the lower and upper jaw segments may be output in a new or updated imaging volume. Accordingly, the new or updated imaging volume can provide an artifact-corrected representation of the upper jaw segment and an artifact-corrected representation of the lower jaw segment, in addition to motion information (e.g., temporal changes over time recorded in a fourth dimension).

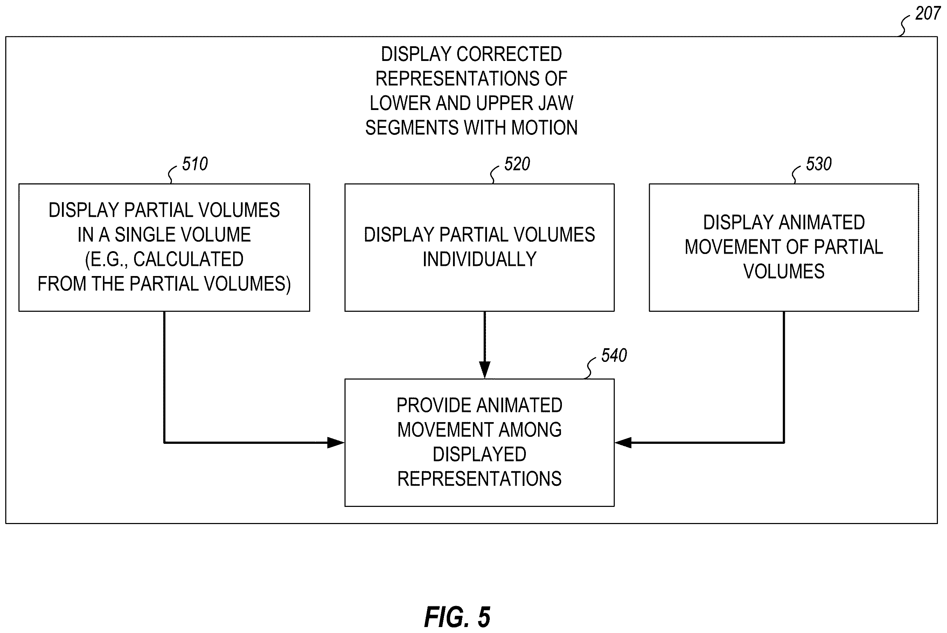

Operation 207 includes displaying corrected representations of the lower and upper jaw segments, including with motion (e.g., with time-based playback or simulation of motion). The display of such motion between the lower and upper jaw segments may be improved as a result of refining the parameters of the motion model, as discussed above. FIG. 5, discussed below, depicts example display techniques to display the respective volumes corresponding to the lower and upper jaw segments. In further examples, other information related to the movement or structures may be displayed or output to a user, such as in a display of the relative movement trajectory of the lower jaw relative to the upper jaw.

Although the preceding operations 202 to 207 refer to examples of motion artifact correction on a reconstructed 3D image volume, other variations to segmentation and motion artifact correction may be based on projection image data. For example, a variation may include an initial segmentation of a sinogram before generating initial partial volumes, followed by motion artifact correction that provides a movement estimate for each partial volume (and adjustment to the segmentation if necessary).

FIG. 3 depicts additional techniques implemented as part of operation 203, for performing segmentation of a lower and upper jaw in a reconstructed volume. These segmentation approaches are performed using an initial implementation of a motion model that defines the expected movement and positions of the upper jaw and the lower jaw, respectively. Operation 203 may provide alternative methods of performing an initial segmentation in operation 310, operation 320, and operation 330, respectively. It will be understood that other variations may be used, including the combination of segmentation techniques, or the refinement of segmentation techniques using some combination or modification of these operations.

Operation 310 provides a first approach for performing segmentation by determining contiguous image areas of rigid structure movement, as applied to the voxels of a reconstructed image volume. This approach may include detecting a deviation in movement of specific voxels as compared with other voxels (e.g., as compared with a majority of the other voxels) in a sub-volume. The segmentation performed on this reconstructed image volume is designed to compensate for distortions caused by the movement. In one example, the segmentation of the image volume compensates for the movement by first reconstructing the volume, considering the movement in the projection geometry, and then performing the segmentation. Without considering the movement in volume reconstruction, the volume would be blurred and encounter severe motion artifacts, and the resulting segmentation would be very limited in quality (or even not possible).

Operation 320 provides a second approach for performing segmentation by determining contiguous areas of rigid structure movement, based on the pixels of one or more projection images. Such pixels can be assigned to several partial volumes. In some examples, the contours of the jaw can be segmented and then transformed to perform pixel segmentation. Additionally, in some examples, landmarks detected in projection images can be used for guidance of the segmentation. It will be understood that an initial reconstruction of the image volume (e.g., in operation 202) does not need to be performed before segmentation is performed on the projection images.

Operation 330 provides a third approach for performing semantic segmentation by using one or more trained models (e.g., trained artificial intelligence models) such as neural networks. The semantic segmentation may be performed on voxels of the image volume or on pixels of one or more projection images. As noted above, the initial reconstruction of the image volume (e.g., in operation 202) does not need to be performed before segmentation is performed on the projection images.

The results of operations 310, 320, and 330 are used to produce an initial segmentation of imaging data (e.g., a segmentation of pixels or voxels) that define segments corresponding to the lower jaw and the upper jaw. Operation 350 includes refining the initial segmentation of this imaging data, based on estimated anatomical conditions of the patient. An example of estimated anatomical conditions may be based on known shapes or areas of the image (e.g., that an upper area of the image represents the upper jaw, while a lower area of the image represents the lower jaw). The refined segmentation based on estimated anatomical conditions can be refined by operations 310, 320, 330, and 350 again. Operation 360 includes optionally refining the segmentation based on user input. The optionally refined segmentation based on user input can be refined by operations 310, 320, 330, and 350 again. The resulting segmentation of this imaging data provides a starting point for motion artifact correction in operation 204.

FIG. 4 depicts additional techniques implemented as part of operation 204, for performing motion artifact correction on two partial volumes of the imaging data (e.g., a first partial volume produced from an upper jaw segment corresponding to the upper jaw and a second partial volume produced from a lower jaw segment corresponding to the lower jaw). Operation 204 may provide alternative methods of performing the motion artifact correction in operation 420, operation 430, and operation 440, respectively. It will be understood that other variations may be used, including the combination of correction techniques, or the refinement of correction techniques using some combination or modification of these operations.

First, at operation 410, an estimation of movement is produced, with separate movement estimated values that correspond to the lower jaw segment and/or the upper jaw segment.

Operation 420 provides a first approach for performing motion artifact correction, by using the estimation of movement for an independent correction of the partial volumes of the imaging data (e.g., the first partial volume corresponding to the upper jaw and the second partial volume corresponding to the lower jaw). Thus, this correction technique is based on an independent estimation of two absolute movements of the upper and lower segments.

Operation 430 provides a second approach for performing motion artifact correction, by using the estimation of movement for a sequential correction of the partial volumes of the imaging data. This may include correcting the first partial volume (e.g., corresponding to the upper jaw), followed by correcting the second partial volume (e.g., corresponding to the lower jaw). A similar approach may involve correcting the partial volume corresponding to the lower jaw followed by correcting the partial volume corresponding to the upper jaw. Thus, this correction technique is based on an estimation of the relative movement between the partial volumes.

Operation 440 provides a third approach for performing motion artifact correction, by using the estimation of movement, in addition to the motion model, for the correction of the partial volumes of the imaging data. Thus, this correction technique is based on a functional dependency of the partial volumes.

In further examples, the optimization of a particular metric of motion artifact correction may be performed on the projection images or in the reconstructed image volume. As will be understood, various approaches for motion artifact correction may include optimizing one or more metrics, because the motion artifact correction may change the underlying shape or size of the anatomical objects (e.g., the upper or lower jaw) portrayed in the reconstructed volume. This may cause the need for reconstructing the partial volume iteratively, if necessary.

Thus, the results of the motion artifact correction on the partial volume(s) can be used to optimize or improve segmentation. Operation 450 provides an optional approach for reconstructing the lower and/or the upper segments, such as by adjusting (e.g., refining) the segmentation of one or both of the segments. Additional motion artifact correction then may be performed by repeating operation 410 and a motion correction operation (e.g., operation 420, 430, or 440).

FIG. 5 depicts additional techniques implemented as part of operation 207, for displaying artifact-corrected representations of the lower and upper jaw segments with motion. Other types of outputs or measurements from the jaw segments may also be provided. Operation 207 may provide alternative methods of displaying the corrected representations in operation 510, operation 520, and operation 530, respectively. It will be understood that other variations may be used, including the combination of display techniques.

At operation 510, all of the partial volumes are displayed in a representation of a single volume, which is produced from the artifact-corrected partial volumes. In other words, this combined volume is calculated from a combination of the partial volume of the upper jaw segment and the partial volume of the lower jaw segment.

At operation 520, the partial volumes are displayed individually, such as with a representation produced from the partial volume of the upper jaw segment or a partial volume of the lower jaw segment.

At operation 530, an animated movement of the partial volumes is displayed. This animated movement may be provided in connection with the display of some functional evaluation of the jaw, and optionally in connection with the display of measured values.

At operation 540, various animated movements (e.g., movement over time) may be presented, as corresponding to the movements originally captured from the patient or corresponding to some movement model. Variations to the displays of the jaw segments and replicated jaw motion may be provided for specific dental or orthodontal applications, such as for the creation of a dental restoration (e.g., to simulate how the restoration will interact with jaw movements and bites), visualization of occlusal contact points (e.g., to simulate how teeth will contact each other during a bite), and the like.

Some variations of displays may include showing animated movement by depicting controlled jaw movements during 3D or 4D data visualizations and manipulations and illustrating aspects of movement relative to the surfaces of the anatomical structures. In addition to displaying animations of jaw movement, these displays may also present parameters or numerical measurements, such as general parameters relating to an amount of bite contact or a range of motion (e.g., how wide the patient's jaw can open). Such parameters may also be used to enhance the polishing or mechanical optimization of a crown shape or finalize a designed shape of a crown or another restoration.

FIG. 6 depicts a representation of an example segmentation of jaw segments into a lower jaw segment 620 and an upper jaw segment 610. This depiction shows how each segment may be defined based on the shape of the rigid anatomical structures captured originally from the x-ray data. Specifically, the segmentation produces the shape of the upper jaw segment 610 based on the shape of the upper jaw 601, and the segmentation produces the shape of the lower jaw segment 620 based on the shape of the lower jaw 602.

FIG. 6 also depicts how the motion between the rigid structures may be based on a specific point or position in the image volume. For instance, movement of the upper jaw segment and the lower jaw segment may pivot on an axis located in area 630. This area 630 may correspond to a general location of the patient's temporomandibular joint. In some examples, the amount of movement of the upper jaw segment and the lower jaw segment may correspond to the application of motion artifact correction, which produces an estimated movement of the corrected volume segment.

Other information related to jaw movement may assist additional digital imaging or diagnostic use cases. For example, after calculating jaw movement for a particular patient, the jaw movement information can be transferred to-or combined with-a digital impression of the patient's dentition obtained by an optical scan (e.g., a surface scan performed on the teeth and the mouth). An optical scan can provide high accuracy in a coordinate system to be correlated to aspects of the x-ray imaging data. Accordingly, extensions of the preceding techniques may include the integration of motion and motion representations into optical scan representations and a variety of other imaging and diagnostic applications.

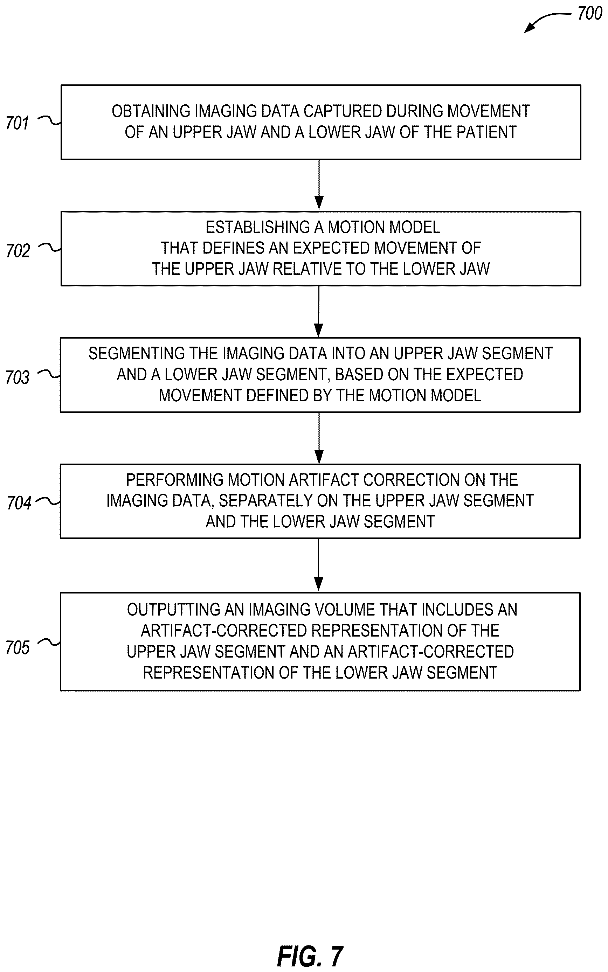

FIG. 7 depicts a flowchart 700 of an example method for generating an imaging volume with captured motion, according to an example.

Operation 701 includes obtaining imaging data that portrays the maxillofacial region of a patient during movement of an upper jaw and a lower jaw of the patient. In an example, the imaging data is CBCT imaging data. For instance, the CBCT imaging data may comprise a plurality of CBCT projection images captured during multiple rotations of a CBCT scanner around the patient. Alternatively, the CBCT imaging data may comprise a three-dimensional imaging volume.

Operation 702 includes establishing a motion model that defines an expected movement of the upper jaw relative to the lower jaw. This motion model may define an expected position, over time, for each of the upper jaw and the lower jaw, corresponding to the expected movement. The parameters of the motion model may be determined or modified based on: one or more landmarks detected in the imaging data; motion of the upper and the lower jaw estimated in motion artifact correction; motion corresponding to a guided jaw movement; motion corresponding to a standard jaw movement; one or more measurements of a tracking system; and/or user input relating to the motion model. In specific examples, the expected movement of the upper jaw relative to the lower jaw is determined or refined based on motion of the upper jaw and the lower jaw estimated in motion artifact correction.

Operation 703 includes segmenting the imaging data into an upper jaw segment and a lower jaw segment, based on the expected movement defined by the motion model. In some examples, the motion model is used to establish the expected movement of the upper jaw segment relative to the lower jaw segment, based on a joint (e.g., a temporomandibular joint) located in part in the upper jaw segment and located in part in the lower jaw segment. In specific examples, the segmenting of the imaging data is based on: segmentation of contiguous image areas of rigid structure among voxels in an image volume of the imaging data, when the image volume is reconstructed considering movement; segmentation of contiguous image areas of rigid structure movement among pixels in projection images of the imaging data; and/or semantic segmentation of the image volume or the projection images, when the semantic segmentation is performed with at least one trained segmentation model. Also in specific examples, the segmenting of the imaging data is refined based on: estimated anatomical conditions of the upper jaw; estimated anatomical conditions of the lower jaw; and/or user input relating to the segmentation.

Operation 704 includes performing motion artifact correction on the imaging data, separately on the upper jaw segment and the lower jaw segment. This motion artifact correction may be performed based on estimated motion corresponding to the lower jaw segment and/or the upper jaw segment, including: independent motion artifact correction of the upper jaw segment and the lower jaw segment that is based on the estimated motion; sequential motion artifact correction of one of the upper jaw segment or the lower jaw segment followed by correction of the other of the upper jaw segment or the lower jaw segment that is based on the estimated motion; and/or motion artifact correction of the upper jaw segment and the lower jaw segment that is based on the estimated motion and the motion model.

Operation 705 includes outputting an imaging volume that includes an artifact-corrected representation of the upper jaw segment and an artifact-corrected representation of the lower jaw segment. In some examples, the outputting includes displaying corrected representations of the upper jaw segment and the lower jaw segment with motion, based on the motion model (e.g., which applies the results of the functional analysis).

The techniques discussed herein may be used to produce or enhance many types of images, including 2D images, 3D images, 4D images (e.g., dynamic CBCT images that depict motion and changes over time as a fourth dimension), and related representations. Such imaging can be used to assist dental treatments, such as restorative treatments, endodontic treatments, periodontal treatments, implant treatments, aligner treatments, orthodontic treatments, and the like. Such images therefore may be used to produce therapeutic and diagnostic information related to teeth, roots, nerve canals, temporomandibular joints, jaw bones, etc. Moreover, the example embodiments described herein are not limited to CBCT imaging techniques but may also be applied to other forms of x-rays and radiographic imaging.

Although the preceding segmentation and artifact correction techniques were described with reference to the movement of a patient's jaw, it will be understood that other types of movements of rigid anatomical structures in CBCT data may be simulated and captured with variations of the present techniques. For example, patient airway movements caused by patient breaths can be identified and corrected with similar methods that are used for reconstructing x-ray images of a rigid anatomical structure. Moreover, the techniques discussed herein are not limited to those in the dental or maxillofacial setting, as other anatomical areas may be captured, segmented, corrected, and represented.

As will be appreciated, the example aspects described herein can be implemented using a single computer or using a computer system that includes multiple computers, each programmed with control logic to perform various of the above-described functions. Embodiments may be implemented in hardware (e.g., circuitry), firmware, software, or any combination thereof. Embodiments may also be implemented as instructions stored on a machine-readable medium, which may be read and executed by one or more processors (e.g., implementations of processor circuitry).

FIG. 8 depicts a block diagram of an example of a computing system 800 (or a suitable apparatus, device, system or machine) upon which any one or more of the techniques (e.g., methods) discussed herein may be performed. In alternative embodiments, the system 800 may operate as a standalone device or may be connected (e.g., networked) to other machines. In a networked deployment, the system 800 may operate in the capacity of a server machine, a client machine, or both in server-client network environments. The system 800 may be a personal computer (PC), a tablet PC, server computer, a mobile telephone, a web appliance, a networked device, or any machine capable of executing instructions (sequential or otherwise) that specify actions to be taken by that machine. Further, while only a single machine is illustrated, the term "machine" shall also be taken to include any collection of machines that individually or jointly execute a set (or multiple sets) of instructions to perform any one or more of the methodologies discussed herein, such as cloud computing, software as a service (SaaS), other computer cluster configurations.

The system 800 may receive a user command by way of any of input device 812, UI navigation device 814, display device 810, or signals from remote systems (e.g., received via network 826). The system 800 may output an indication that an action has been performed by way of any of display device 810 and signal generation device 818. The system 800 also may provide output of the generated imaging volume in a machine readable medium 822 based on the techniques to generate the imaging volume from an artifact-corrected representation of an upper jaw segment and an artifact-corrected representation of a lower jaw segment as described herein. The system 800 may be used to implement the operations performed by the method of generating an imaging volume of a maxillofacial region (e.g., as discussed with reference to FIG. 7), and related data processing operations involved in processing, reconstructing, segmenting, modeling, refining, representing, and visualizing related data.

System 800 (e.g., computer system) may include processing circuitry such as a hardware processor 802 (e.g., a central processing unit (CPU), a graphics processing unit (GPU), a hardware processor core, field programmable gate array (FPGA), or any combination thereof), a main memory 804 and a static memory 806, some or all of which may communicate with each other via an interlink (e.g., bus) 830. The system 800 may further include a display device 810, an input device 812 (e.g., a keyboard or other alphanumeric input device), and a user interface (UI) navigation device 814 (e.g., a mouse). In an example, the display device 810, input device 812, and UI navigation device 814 may be integrated into a touch screen display. The system 800 may additionally include a mass storage device 816 (e.g., drive unit or other similar storage device or unit), a signal generation device 818 (e.g., a speaker), a network interface device 820 connected to a network 826, and one or more sensors 808. The system 800 may include an output controller 828, such as a serial (e.g., universal serial bus (USB), parallel, or other wired or wireless (e.g., infrared (IR), near field communication (NFC), etc.) connection to communicate or control one or more peripheral devices (e.g., a printer, card reader, etc.).

The mass storage device 816 may include a machine readable medium 822 on which is stored one or more sets of data structures or instructions 824 (e.g., software) embodying or used by any one or more of the techniques or functions described herein. The instructions 824 may also reside, completely or at least partially, within the main memory 804, within static memory 806, or within the hardware processor 802 during execution thereof by the system 800. In an example, one or any combination of the hardware processor 802, the main memory 804, the static memory 806, or the mass storage device 816 may constitute machine readable media.

While the machine readable medium 822 is illustrated as a single medium, the term "machine readable medium" may include a single medium or multiple media (e.g., a centralized or distributed database, and/or associated caches and servers) configured to store the one or more instructions 824. The term "machine readable medium" may include any medium that is capable of storing, encoding, or carrying instructions for execution by the system 800 and that cause the system 800 to perform any one or more of the techniques of the present disclosure, or that is capable of storing, encoding, or carrying data structures used by or associated with such instructions. Non-limiting machine-readable medium examples may include solid-state memories, and optical and magnetic media. Specific examples of machine-readable media (e.g., one or more non-transitory storage medium) may include: read only memory (ROM); random access memory (RAM); non-volatile memory, such as semiconductor memory devices (e.g., Electrically Programmable Read-Only Memory (EPROM), Electrically Erasable Programmable Read-Only Memory (EEPROM)) and flash memory devices; magnetic disks, such as internal hard disks and removable disks; magneto-optical disks; and CD-ROM and DVD-ROM disks.

Examples, as described herein, may include, or may operate by, logic or a number of components, or mechanisms, including in circuitry. Circuitry includes a collection of circuits implemented in tangible entities that include hardware (e.g., simple circuits, gates, logic, etc.). Circuitry membership may be flexible over time and underlying hardware variability. Circuitry includes members that may, alone or in combination, perform specified operations when operating. In an example, hardware of the circuitry may be immutably designed to carry out or conduct a specific operation (e.g., hardwired). In an example, the hardware of the circuitry may include variably connected physical components (e.g., execution units, transistors, simple circuits, etc.) including a computer readable medium physically modified (e.g., magnetically, electrically, moveable placement of invariant massed particles, etc.) to encode instructions of the specific operation. The instructions enable embedded hardware (e.g., the execution units or a loading mechanism) to create members of the circuitry in hardware via the variable connections to carry out or conduct portions of the specific operation when in operation. Accordingly, the computer readable medium is communicatively coupled to the other components of the circuitry when the device is operating. In an example, any of the physical components may be used in more than one member of more than one circuitry unit. For example, under operation, execution units may be used in a first circuit of a first circuitry unit at one point in time and reused by a second circuit in the first circuitry unit, or by a third circuit in a second circuitry unit at a different time.

Embodiments thus may be implemented in hardware (e.g., circuitry), firmware, software, or any combination thereof. Embodiments may also be implemented as instructions stored on a machine-readable medium, which may be read and executed by one or more processors. Further, firmware, software, routines, instructions may be described herein as performing certain actions. However, it should be appreciated that such descriptions are merely for convenience and that such actions in fact result from computing devices, processors, controllers, or other devices executing the firmware, software, routines, instructions, etc. Further, any of the implementation variations may be carried out by a general-purpose computer.

The various components described herein may be referred to as "modules," "units," "devices", "functionality", or similar terms. Such components may be implemented via any suitable combination of hardware and/or software components as applicable and/or known to achieve their intended respective functionality. This may include mechanical and/or electrical components, processors, processing circuitry, or other suitable hardware components, in addition to or instead of those discussed herein. Such components may be configured to operate independently, or configured to execute instructions or computer programs that are stored on a suitable computer-readable medium. Regardless of the particular implementation, such components, as applicable and relevant, may alternatively be referred to herein as "circuitry," "controllers," "processors," or "processing circuitry," or using alternative terms as noted herein.

Additional aspects and features of the present disclosure are set forth in the following numbered examples:

Example 1 is a method of generating an imaging volume of a maxillofacial region, comprising: obtaining imaging data that portrays the maxillofacial region of a patient during movement of an upper jaw and a lower jaw of the patient; establishing a motion model that defines an expected movement of the upper jaw relative to the lower jaw; segmenting the imaging data into at least an upper jaw segment corresponding to the upper jaw and a lower jaw segment corresponding to the lower jaw, based on the expected movement defined by the motion model; performing motion artifact correction on the imaging data, wherein the motion artifact correction is performed separately on the upper jaw segment and the lower jaw segment; and outputting an imaging volume that includes an artifact-corrected representation of the upper jaw segment and an artifact-corrected representation of the lower jaw segment.

In Example 2, the subject matter of Example 1 optionally includes wherein the imaging data is cone-beam computed tomography (CBCT) imaging data.

In Example 3, the subject matter of Example 2 optionally includes wherein the CBCT imaging data comprises a plurality of CBCT projection images captured during multiple rotations of a CBCT scanner around the patient.

In Example 4, the subject matter of any one or more of Examples 2-3 optionally include wherein the CBCT imaging data comprises a three-dimensional imaging volume.

In Example 5, the subject matter of any one or more of Examples 1-4 optionally include wherein the motion model defines an expected position over time, for each of the upper jaw and the lower jaw, corresponding to the expected movement.

In Example 6, the subject matter of Example 5 optionally includes wherein parameters of the motion model are determined or refined based on at least one of: one or more landmarks detected in the imaging data; motion of the upper jaw and the lower jaw estimated in motion artifact correction; motion corresponding to a guided jaw movement; motion corresponding to a standard jaw movement; one or more measurements of a tracking system; or user input relating to the motion model.

In Example 7, the subject matter of any one or more of Examples 1-6 optionally include wherein the expected movement is determined or refined based on motion of the upper jaw and the lower jaw estimated in motion artifact correction.

In Example 8, the subject matter of any one or more of Examples 1-7 optionally include wherein the motion model is used to establish the expected movement of the upper jaw segment relative to the lower jaw segment, based on a joint located in part in the upper jaw segment and located in part in the lower jaw segment.

In Example 9, the subject matter of any one or more of Examples 1-8 optionally include wherein the segmenting of the imaging data is based on: segmentation of contiguous image areas of rigid structure among voxels in an image volume of the imaging data, wherein the image volume is reconstructed considering movement; segmentation of contiguous image areas of rigid structure movement among pixels in projection images of the imaging data; or semantic segmentation of the image volume or the projection images, wherein the semantic segmentation is performed with at least one trained segmentation model.

In Example 10, the subject matter of any one or more of Examples 1-9 optionally include wherein the segmenting of the imaging data is refined based on at least one of: estimated anatomical conditions of the upper jaw; estimated anatomical conditions of the lower jaw; or user input relating to the segmentation.

In Example 11, the subject matter of any one or more of Examples 1-10 optionally include wherein the motion artifact correction is performed based on an estimated motion corresponding to at least one of the lower jaw segment or the upper jaw segment, and wherein: independent motion artifact correction of the upper jaw segment and the lower jaw segment is based on the estimated motion; sequential motion artifact correction of one of the upper jaw segment or the lower jaw segment followed by correction of the other of the upper jaw segment or the lower jaw segment is based on the estimated motion; or motion artifact correction of the upper jaw segment and the lower jaw segment is based on the estimated motion and the motion model.

In Example 12, the subject matter of any one or more of Examples 1-11 optionally include wherein outputting the imaging volume includes displaying corrected representations of the upper jaw segment and the lower jaw segment with motion, based on the motion model.

In Example 13, the subject matter of any one or more of Examples 1-12 optionally include outputting audible or visible instructions to the patient during an acquisition of the imaging data, wherein the audible or visible instructions guide the patient to perform the movement of the upper jaw or the lower jaw.

In Example 14, the subject matter of any one or more of Examples 1-13 optionally include wherein the imaging volume is a four-dimensional volume that portrays the movement of the upper jaw and the lower jaw over time.

Example 15 is at least one machine-readable medium, including instructions, which when executed by processing circuitry, cause the processing circuitry to perform any of the methods of Examples 1 to 12, to generate an imaging volume of the maxillofacial region with motion.

Example 16 is an x-ray system, comprising: an x-ray source and an x-ray detector for acquiring imaging data from the maxillofacial region of a patient, the imaging data acquired during movement of an upper jaw relative to the lower jaw of the patient; and a computing unit for performing any of the methods of Examples 1 to 12, to generate an imaging volume of the maxillofacial region with motion.

Example 17 is a computer system, comprising: memory storing imaging data from the maxillofacial region of a patient, the imaging data acquired during movement of an upper jaw relative to the lower jaw of the patient; and at least one processor configured to perform any of the methods of Examples 1 to 12, to generate an imaging volume of the maxillofacial region with motion.

The various embodiments described above have been presented by way of example and not limitation. It will be apparent to persons skilled in the relevant art(s) that various changes in form and detail can be made therein (e.g., different hardware, communications protocols, and the like) without departing from the scope of the present embodiments. In addition, it should be understood that the attached drawings, which highlight functionality described herein, are presented as illustrative examples. The architecture of the present aspects is sufficiently flexible and configurable, such that it can be utilized and navigated in ways other than that shown in the drawings.

## Claims

1. A method of generating an imaging volume of a maxillofacial region, comprising:
obtaining imaging data that portrays the maxillofacial region of a patient during movement of an upper jaw and a lower jaw of the patient;
establishing a motion model that defines an expected movement of the upper jaw relative to the lower jaw;
segmenting the imaging data into at least an upper jaw segment corresponding to the upper jaw and a lower jaw segment corresponding to the lower jaw, based on the expected movement defined by the motion model;
performing motion artifact correction on the imaging data, wherein the motion artifact correction is performed separately on the upper jaw segment and the lower jaw segment; and
outputting an imaging volume that includes an artifact-corrected representation of the upper jaw segment and an artifact-corrected representation of the lower jaw segment.

2. The method of claim 1, wherein the imaging data is cone-beam computed tomography (CBCT) imaging data.

3. The method of claim 2, wherein the CBCT imaging data comprises a plurality of CBCT projection images captured during multiple rotations of a CBCT scanner around the patient.

4. The method of claim 2, wherein the CBCT imaging data comprises a three-dimensional imaging volume.

5. The method of claim 1, wherein the motion model defines an expected position over time, for each of the upper jaw and the lower jaw, corresponding to the expected movement.

6. The method of claim 5, wherein parameters of the motion model are determined or refined based on at least one of:
one or more landmarks detected in the imaging data;
motion of the upper jaw and the lower jaw estimated in motion artifact correction;
motion corresponding to a guided jaw movement;
motion corresponding to a standard jaw movement;
one or more measurements of a tracking system; or
user input relating to the motion model.

7. The method of claim 1, wherein the expected movement is determined or refined based on motion of the upper jaw and the lower jaw estimated in motion artifact correction.

8. The method of claim 1, wherein the motion model is used to establish the expected movement of the upper jaw segment relative to the lower jaw segment, based on a joint located in part in the upper jaw segment and located in part in the lower jaw segment.

9. The method of claim 1, wherein the segmenting of the imaging data is based on:
segmentation of contiguous image areas of rigid structure among voxels in an image volume of the imaging data, wherein the image volume is reconstructed considering movement;
segmentation of contiguous image areas of rigid structure movement among pixels in projection images of the imaging data; or
semantic segmentation of the image volume or the projection images, wherein the semantic segmentation is performed with at least one trained segmentation model.

10. The method of claim 1, wherein the segmenting of the imaging data is refined based on at least one of:
estimated anatomical conditions of the upper jaw;
estimated anatomical conditions of the lower jaw; or
user input relating to the segmentation.

11. The method of claim 1, wherein the motion artifact correction is performed based on an estimated motion corresponding to at least one of the lower jaw segment or the upper jaw segment, and wherein:
independent motion artifact correction of the upper jaw segment and the lower jaw segment is based on the estimated motion;
sequential motion artifact correction of one of the upper jaw segment or the lower jaw segment followed by correction of the other of the upper jaw segment or the lower jaw segment is based on the estimated motion; or
motion artifact correction of the upper jaw segment and the lower jaw segment is based on the estimated motion and the motion model.

12. The method of claim 1, wherein outputting the imaging volume includes displaying corrected representations of the upper jaw segment and the lower jaw segment with motion, based on the motion model.

13. The method of claim 1, further comprising:
outputting audible or visible instructions to the patient during an acquisition of the imaging data, wherein the audible or visible instructions guide the patient to perform the movement of the upper jaw or the lower jaw.

14. At least one machine-readable medium, including instructions, which when executed by processing circuitry, cause the processing circuitry to perform any of the methods of claims 1 to 13, to generate an imaging volume of the maxillofacial region with motion.

15. An x-ray system, comprising:
an x-ray source and an x-ray detector for acquiring imaging data from the maxillofacial region of a patient, the imaging data acquired during movement of an upper jaw relative to the lower jaw of the patient; and
a computing unit for performing any of the methods of claims 1 to 13, to generate an imaging volume of the maxillofacial region with motion.
